# EUROPEAN PATENT APPLICATION

(11) **EP 1 186 286 A1**
(43) Date of publication of application: **13.03.2002**
(21) Application number: 00935604.9
(22) Date of filing: 09.06.2000
(51) Int. Cl.: A61K 7/00

(54) **HEAT-EVOLVING COSMETICS**

(30) Priority: 11.06.1999 JP 16515199; 01.07.1999 JP 18751499; 01.07.1999 JP 18752099; 06.09.1999 JP 25133899; 25.01.2000 JP 2000015546; 25.01.2000 JP 2000015547; 25.01.2000 JP 2000015548
(71) Applicant: Kanebo Limited, Sumida-ku, Tokyo 131-0031 (JP)
(72) Inventor: SAITO, Masato Kanebo, Limited, Odawara-shi Kanagawa 250-0002 (JP); TEZUKA, Keizo, Ashigarakami-gun Kanagawa 258-0024 (JP)
(74) Representative: Henkel, Feiler, Hänzel
(86) International application number: JP0003752
(87) International publication number: WO0076462

(57) **Abstract**

A heat-generating cosmetic comprising
(a) a polyhydric alcohol and/or a polyoxyalkylene (having 2 or 3 carbon atoms)-glycol adduct, which generates heat when being in contact with water,
(b) one or more compounds selected from the group consisting of silicic acid anhydride, silicic acid hydrate, synthetic hydrotalcite and synthetic calcined hydrotalcite, and
(c) a thickening agent
and which is substantially non-aqueous,
Or, a heat-generating cosmetic comprising
(a) a polyoxyalkylene (having 2 or 3 carbon atoms)glycol adduct, which generates heat in contact with water,
(b) zeolite, and
(c) a thickening agent.
and which is substantially non-aqueous.

These heat-generating cosmetics feature long heat generating action, not drip from the skin, and are excellent in feeling (e.g. giving no clammy feeling).

## Description

### TECHNICAL FIELD

The present invention relates to a heat-generating cosmetic, which generates heat when brought into contact with water.

### BACKGROUND ART

Various materials for providing heat-generating action to cosmetics to give one pleasant feelings when the cosmetics are applied, enhance skin hygiene and skin functions, etc., have been suggested in the past. For example, a cosmetic utilizing the exothermic reaction of alkylene glycol, etc. in contact with water (Japanese Prov. Patent Publn. No.75909/1982), a cosmetic utilizing the exothermic reaction of polyethylene glycol and activated zeolite in contact with water (Japanese Prov. Patent Publn. No.100411/1994), and also a pack, etc. utilizing the exothermic reaction of exsiccated gypsum in contact with water (Japanese Prov. Patent Publns. No.114506/1982, No.94905/1985, No.30704/1987, No.54308/1988) are disclosed.

However, heat-generating cosmetics by conventional methods have disadvantages in utilities or sensations; for example, their heat-generating action reduces with the lapse of time, though they are strong at the time of application, they drip off when applied to the skin, or they give one clammy feeling at the time of application. Furthermore, exsiccated gypsum, etc. become strongly alkaline, thus they are undesirable for the skin; materials formulated by combining polyethylene glycol and activated zeolite are inferior in wash-away ability after application.

An object of the present invention is to provide a heat-generating cosmetic, which keeps its heat-generating action, does not drip off from the skin, gives one no clammy feeling, etc., thus giving an excellent feeling.

Furthermore, an object of the present invention is to provide a cosmetic, which has an excellent wash-away ability, feeling, etc., and also keeps its heat-generating action for a long time.

### SUMMARY OF THE INVENTION

The inventors studied earnestly to solve the above problems and as a result, they have found that a heat-generating cosmetic comprising
(a) a polyhydric alcohol and/or a polyoxyalkylene (having 2 or 3 carbon atoms)-glycol adduct, which generates heat in contact with water,
(b) one or more compounds selected from the group consisting of silicic acid anhydride, silicic acid hydrate, synthetic hydrotalcite and synthetic calcined hydrotalcite, and
(c) a thickening agent,
and which is substantially non-aqueous,
or, a heat-generating cosmetic comprising
(a) a polyoxyalkylene (having 2 or 3 carbon atoms)glycol adduct, which generates heat in contact with water,
(b) zeolite, and
(c) a thickening agent,
and which is substantially non-aqueous,
could attain the above object.

Namely, the present invention relates to a heat-generating cosmetic comprising
(a) a polyhydric alcohol and/or a polyoxyalkylene (having 2 or 3 carbon atoms)-glycol adduct, which generates heat in contact with water,
(b) silicic acid anhydride and/or silicic acid hydrate,
(c) a thickening agent,
and which is substantially non-aqueous.

Or the present invention relates to a heat-generating cosmetic comprising
(a) a polyoxyalkylene (having 2 or 3 carbon atoms)glycol adduct, which generates heat in contact with water,
(b) zeolite, and
(c) a thickening agent, and which is substantially non-aqueous.

Also, one of the preferred embodiments of the present invention relates to the above heat-generating cosmetic, wherein a polyhydric alcohol or a polyoxyalkylene (having 2 or 3 carbon atoms)-glycol adduct is selected from polyethylene glycol, 1,3-butylene glycol, glycerol, polyoxyethylene glyceryl ether and polyoxyalkylene-modified organopolysiloxane.

Also, another preferred embodiment of the present invention relates to the above heat-generating cosmetic, wherein the thickening agent is at least one compound selected from the group consisting of hydroxypropyl cellulose, aluminum starch octenylsuccinate, synthetic aluminum silicate and kaolin.

Further, still another preferred embodiment of the present invention relates to the above heat-generating cosmetic, which further comprises (d) sodium polyacrylate powder.

### BEST MODE FOR CARRYING OUT THE INVENTION

According to the present invention, silicic acid anhydride, silicic acid hydrate, synthetic hydrotalcite or a calcined product thereof is used together with a polyhydric alcohol or a polyoxyalkylene (having 2 or 3 carbon atoms)-glycol adduct which generates heat in contact with water, to control heat-generation and to provide moisture retention. Also, according to the present invention, by using a polyoxyalkylene (having 2 or 3 carbon atoms in alkylene group)-glycol adduct which generates heat in contact with water and zeolite together, heat-generation of zeolite is controlled so that the heat-generating action can be sustained for a long time, and also water wash-away ability after the heat-generating cosmetic is applied to the skin is excellent as compared with those using mere polyoxyethylene glycol, glycerol, etc. in combination to utilize hydration heat thereof.

The Polyhydric alcohols or the polyoxyalkylene (having 2 or 3 carbon atoms)-glycol adducts, which generate heat in contact with water, used in the present invention, are for example polyhydric alcohol such as ethylene glycol, diethylene glycol, dipropylene glycol, polyethylene glycol, polypropylene glycol, copolymer of polyoxyethylene and polyoxypropylene, 1,3-butylene glycol, propylene glycol, glycerol and polyglycerol, etc. Further, as the polyoxyalkylene (having 2 or 3 carbon atoms)-glycol adducts, there may be mentioned for example, a polyoxyethylene alkyl (having 12 to 14 carbon atoms) ether such as polyoxyethylene glyceryl ether, polyoxypropylene glyceryl ether, polyoxyethylene polyoxypropylene glyceryl ether and polyoxyethylene lauryl ether; a polyoxyalkylene aliphatic ester such as polyethylene glycol monolaurate, polypropylene glycol laurate and polyethylene glycol dilaurate; polyoxyethylene methyl glucoside and polyoxyalkylene-modified organopolysiloxane. The total number of additional units of one or more ethylene oxide or propylene oxide in the adduct is preferably 10 to 100. They are used alone or in combination with two or more of them. Among them, polyethylene glycol (as to the number average molecular weight, 100 to 800 is preferred), 1,3-butylene glycol, glycerol, polyoxyethylene glyceryl ether or polyoxyalkylene-modified organopolysiloxane is preferred in order to attain the object of the present invention. It is also particularly preferable to use two or more of them to keep heat generation at a pleasant temperature. Since the heat-generating cosmetic of the present invention has viscosity, preferably polyoxyalkylene-modified organopolysiloxane is used together to obtain a deaerating effect.

According to the present invention, by using the polyoxyalkylene (having 2 or 3 carbon atoms)-glycol adduct which generates heat in contact with water and zeolite together, heat-generation of zeolite is controlled so that heat-generating action can be sustained for a long time, and also water wash-away ability after the heat-generating cosmetic is applied to the skin is excellent as compared with those using mere polyoxyethylene glycol, glycerol, etc. in combination to utilize hydration heat thereof.

As to the amount of the polyhydric alcohol and/or the polyoxyalkylene (having 2 or 3 carbon atoms)-glycol adduct, when silicic acid anhydride, silicic acid hydrate, synthetic hydrotalcite or calcined synthetic hydrotalcite is formulated to the heat-generating cosmetic, it is preferably 20 to 95 % by weight based on the total weight of the heat-generating cosmetic, particularly preferably 40 to 80 % by weight. Also, as to the total amount of the polyoxyalkylene (having 2 or 3 carbon atoms in alkylene group)-glycol adduct, when zeolite is formulated to the heat-generating cosmetic, it is preferably 5.0 to 70.0 % by weight based on the total amount of the heat-generating cosmetic, particularly preferably 10.0 to 60.0 % by weight.

Silicic acid anhydride and silicic acid hydrate used in the present invention are the materials that generate heat of cohesion when the powder thereof are in contact with water; those which are used together with the above polyhydric alcohol or polyoxyalkylene (having 2 or 3 carbon atoms)-glycol adduct to control heat-generating action; also those formulated to decrease the clammy feeling caused by the above polyhydric alcohol or polyoxyalkylene (having 2 or 3 carbon atoms)-glycol adduct. SYLOPURE series (silicic acid anhydride, Fuji Silysia Chemical Ltd.), SYLYSIA series (silicic acid hydrate, Fuji Silysia Chemical Ltd.), etc. are commercially available. Among them, SYLOPURE 35, silicic acid anhydride calcined at temperatures higher than 600 °C is particularly preferable since its heat-generation property is enough.

As to the amount of the silicic acid anhydride and/or the silicic acid hydrate, it is preferably 5 to 40 % by weight, particularly preferably 10 to 20 % by weight based on the total amount of the heat-generating cosmetic.

The synthetic hydrotalcite used in the present invention is a basic hydrate of magnesium carbonate and aluminum carbonate, which has a chemical formula such as Mg₆Al₂(OH)₁₆CO₃ · ₄H₂O, Mg_{4.5}Al₂(OH)₁₃CO₃ · 3.5H₂O, Mg_{4.3}Al₂(OH)_{12.6}CO₃ · mH₂O, including KYOWAAD 500, 1000 and DHT-4A commercially available from Kyowa Chemical Industry Co., Ltd. Synthetic calcined hydrotalcite is a material that synthetic hydrotalcite is calcined to eliminate H₂O and CO₂, having a chemical formula such as Mg_{0.7}Al_{0.3}O_{1.15}, and there may be mentioned for example, KYOWAAD 2000 commercially available from Kyowa Chemical Industry Co.,Ltd. etc. Among them, KYOWAAD 2000, etc., which is a calcined product having a good heat-generating property and which is gentle to skin due to its spherical powder form, is particularly preferable. The powder thereof have properties of generating heat of cohesion when they are in contact with water.

As to the total amount of the synthetic hydrotalcite and/or the calcined product thereof, it is preferably 1 to 50 % by weight, particularly preferably 3 to 20 % by weight based on the total weight of the heat-generating cosmetic.

Zeolite used in the present invention is a material which generates heat of hydration when blended with water, and synthetic zeolite powder, for example (1-x)Na₂O · xK₂O · Al₂O₃ · 2SiO₂(x ≧ 0.3) (synthetic zeolite 3A type); Na₂O · Al₂O₃ 2SiO₂(synthetic zeolite 4A type); (1-x)Na₂O · xCaO · Al₂O₃ · 2SIO₂(x ≧ 0.7)(synthetic zeolite 5A type) are preferable. As to the particle size distribution of the synthetic zeolite, 0.15mm or less is preferred, and such products, there may be mentioned Zeolum A-3 powder, Zeolum A-4 powder and Zeolum A-5 powder commercially available from TOSOH Corporation, and others available from UNION SHOWA K.K.

As to the amount of zeolite, it is preferably 1.0 to 50.0 % by weight, particularly preferably 10.0 to 40.0 % by weight based on the total weight of the heat-generating cosmetic.

As to the thickening agents used in the present invention, there may be mentioned organic polymeric compounds such as carboxymethyl cellulose, hydroxymethyl cellulose, hydroxyethyl cellulose, hydroxypropyl cellulose, polyvinyl pyrrolidone; polyvinyl alcohol, carrageenan, xanthan gum, starch derivatives, e.g. aluminum starch octenylsuccinate, inorganic compounds such as kaolin, talc, titanium oxide, aluminum magnesium silicate and synthetic aluminum silicate. They are used alone or in combination of two or more of them. Particularly, hydroxypropyl cellulose, aluminum starch octenylsuccinate, synthetic aluminum silicate and kaolin are preferred, since they are well miscible with the other ingredients, to give an uniform system and suitable viscosity. The heat-generating cosmetic is preferably used in viscosity of 10,000 to 150,000 cps, particularly preferably 30,000 to 100,000 cps, since its heat-generating action can be sustained for a long time, it is easy to apply to the skin, it is good in feeling and also it drips off only to a slight degree. It is particularly preferable to use the above organic polymeric compounds and the above inorganic compounds together to obtain the viscosity in the above range, also the system is uniform when heat generated. The above viscosity is a value obtained by using a B type rotational viscometer at 25 °C.

The thickening agent is used preferably in an amount of 0.5 to 30 % by weight, in particular 3 to 15 % by weight when silicic acid anhydride, silicic acid hydrate or synthetic hydrotalcite is foumulated for the heat-generating cosmetic, while it is used preferably in an amount of 0.5 to 60.0 % by weight, particularly preferably 3.0 to 40.0 % by weight when zeolite is formulated for the heat-generating cosmetic.

In the present invention, besides the polyhydric alcohol and/or the polyoxyalkylene (having 2 or 3 carbon atoms)-glycol adduct, silicic acid anhydride, silicic acid hydrate, synthetic hydrotalcite, calcined synthetic hydrotalcite or zeolite, and the thickening agent, the cosmetic comprises further sodium polyacrylate powder in order to improve the clammy feeling caused by the polyhydric alcohol, the polyoxyalkylene (having 2 or 3 carbon atoms)-glycol adduct and the thickening agent; skin functions are enhanced by massage action brought by sodium polyacrylate working as scrub under a warm temperature; optimum heat-generating effects are obtained by sodium polyacrylate powder mixing the cosmetic and water uniformly on the skin. In particular, it is preferable to color the sodium polyacrylate powder in the shade different from the shade of a surrounding heat-generating cosmetic, since it can be observed with eyes that the powder collapse at the time of application so it can be the guide with the lapse of time. The sodium polyacrylate powder has preferably a particle size of 1 to 80 µm, also is used preferably in an amount of 0.05 to 2.0 % by weight.

In the present invention, other ingredients such as thermal agents, oils, preservatives, pigments, dyes, chelating agents, refrigerants, surfactants, antiphlogistic agents, astringents, cell-activating agents, slimming agents, whitening agents, sebum secretion depressing agents, depilatories, antioxidants, perfumes and the like are properly formulated for the cosmetic depending on the purpose of uses. In particular, it is preferable to formulate agents for enhancing skin functions such as extracts of plants and animals, yeast extracts or vitamins, since their thermal effects strengthen the action of the other ingredients enhancing skin functions. Also, it is preferable to use thermal agents such as tincture of capsicum, extract of capsicum, tincture of ginger, extracts of ginger, capsaicin and derivatives thereof, derivatives of isovanillin, tocopherols, nicotinic acids, alkyl ether of vanillyl alcohol (another name: vanillyl-n-alkyl ether, having 3 to 6 carbon atoms in the alkyl group, in particular the substance described in Japanese Prov. Patent Publn. No.292711/1987, etc.) together in 0.001 to 1.0 % by weight. In the present invention, the cosmetic is required to be substantially non-aqueous, that is to be formulated by no water, in order to obtain heat-generating action only when it is applied.

The heat-generating cosmetic of the present invention is used for preparations for packs, massaging, shaving, depilation, face-washing, hair-treatment, or hair-washing and the like, and is adapted for use in the form of cream, gel, sheet (applied to base material), etc. It is particularly preferable for massaging preparations for packs that are washed away. As to the way of using it, for example a face etc. is wetted by water or lotion, and then the heat-generating cosmetic of the invention is applied, the heat-generating cosmetic and water are mixed together to generate heat while massaging the skin with fingers, then they can be washed away with water or are removed after drying.

Hereinbelow, the present invention is described in more detail by referring to Examples, but these Examples are not at all limitative of the present invention.

As to the evaluation of the heat-generating cosmetic, the heat-generating cosmetic was used by 10 panelists and was examined oraganoleptically regarding each evaluation point to evaluate by the following evaluation criteria 1, etc. Further, the durability of heat generated was evaluated by whether the effects were sustained for 5 minutes or not.

### [organoleptic evaluation criteria 1]

- ⓞ :: 9 to 10 persons answered "good"
- ○ :: 6 to 8 persons answered "good"
- Δ :: 3 to 5 persons answered "good"
- × :: 0 to 2 persons answered "good"

### Examples 1 to 3, comparative Example 1

The washable heat-generating packs were prepared according to the formulations shown in the following Table 1, and the obtained packs were evaluated by the above organoleptic evaluation method. The results are given in Table 1. Amounts of the ingredients are given by % by weight (the same applies hereinafter).

**Table 1**

| Ingredients | Example1 | Example2 | Example3 | Comp. Example1 |
|---|---|---|---|---|
| Polyethylene glycol (number average molecular weight: 200) | 20.0 | 20.0 | 20.0 | 20.0 |
| Polyethylene glycol (number average molecular weight: 400) | 20.0 | 20.0 | 20.0 | 20.0 |
| 1,3-butylene glycol | 15.0 | 15.0 | 10.0 | 15.0 |
| Glycerol | 15.0 | 15.0 | 15.0 | 15.0 |
| Silicic acid anhydride *1 | 5.0 | 5.0 | 5.0 | - |
| Silicic acid hydrate *2 | 10.0 | 10.0 | 5.0 | - |
| Hydroxypropyl cellulose | 0.6 | 0.6 | 0.6 | 0.6 |
| Kaolin | reminder | reminder | reminder | reminder |
| Aluminum starch octenylsuccinate | 2.0 | 2.0 | 2.0 | 2.0 |
| Synthetic sodium silicate | 1.0 | 1.0 | 1.0 | 1.0 |
| Sodium polyacrylate (blue colored powder mean particle diameter: 10 µm) | - | 1.0 | 1.0 | - |
| Silicone oil*3 | 0.2 | 0.2 | 0.2 | 0.2 |
| Witch hazel extract (1.3-BG extract) | 0.2 | 0.2 | 0.2 | - |
| Perfume | 0.1 | 0.1 | 0.1 | 0.1 |
| Viscosity(cps, 25°C) | 10,000 | 20,000 | 84,000 | 3,000 |
| Clammy feeling at the time of application | ○ | ⓞ | ⓞ | × |
| Dripping after application | ○ | ⓞ | ⓞ | Δ |
| Warm feeling and durability | ○ | ○ | ⓞ | Δ |

| | | | | |
|---|---|---|---|---|
| *1 SYLOPURE 35(calcined)/ SYLOPURE 25(1/1)(Fuji Silysia Chemical Ltd., mean particle diameter:7 µm) | | | | |
| *2 SYLYSIA 770(Fuji Silysia Chemical Ltd., mean particle diameter: 6 µm) | | | | |
| *3 polyoxyethylene-modified organopolysiloxane (Shin-Etsu Chemical Co., Ltd., silicone KF-351A, number of additional ethylene oxide units =11) | | | | |

As can be seen from the results given in Table 1 lower column, Examples 1 to 3 according to the present invention indicated durable heat-generating action, no dripping from the skin, and no clammy feeling. Especially Examples 2 and 3 proved excellent. Also, each example showed excellent pack effects, by giving moisture to the skin as compared with the comparative examples. Furthermore, the heat-generating cosmetic in each example had a pH of 5 to 7 and was safe giving no trouble to the skin.

### Example 4

Washable heat-generating packs were prepared according to the formulation shown in Table 2, and obtained packs were evaluated by the above organoleptic evaluation method. The results are given in Table 2.

**Table 2**

| Ingredients | Example4 |
|---|---|
| Polyethylene glycol (number average molecular weight:200) | 20.0 |
| Polyethylene glycol (number average molecular weight:400) | 20.0 |
| Polyoxyethylene glyceryl ether *4 | 15.0 |
| Glycerol | 15.0 |
| Silicic acid anhydride *5 | 15.0 |
| Hydroxypropyl cellulose | 0.6 |
| Kaolin | reminder |
| Aluminum starch octenylsuccinate | 2.0 |
| Synthetic sodium silicate | 1.0 |
| Sodium polyacrylate (mean particle diameter: 10 µm) | 1.0 |
| Silicone oil*3 | 0.2 |
| Witch hazel extract (1,3-BG extract) | 0.2 |
| Sage leaf extract (1,3-BG extract) | 1.0 |
| Asebiol (ASEBIOL BT; trade name) *6 | 1.0 |
| Perfume | 0.1 |
| Viscosity (cps, 25°C) | 83,000 |
| Clammy feeling at the time of application | ⓞ |
| Dripping after application | ⓞ |
| Warm feeling and durability | ⓞ |

| | |
|---|---|
| *3 polyoxyethylene-modified organopolysiloxane (Shin-Etsu Chemical Co.,Ltd. , silicone KF-351A, number of additional ethylene oxide units= 11) | |
| *4 UNIOX G-1200 (total E.O.= 26, NOF CORPORATION) | |
| *5 SYLOPURE 35 (calcined) (Fuji Silysia Chemical Ltd.) | |
| *6 LABORATOIRES SEROBIOLOGIQUES (hydrolyzed yeast/pyridoxine/ niacinamide/glycerol/panthenol/propylene glycol/allantoin/biotin included) | |

### Examples 5 to 7, Comparative Example 2

Washable heat-generating packs were prepared according to the formulations shown in Table 3, and the obtained packs were oraganoleptically evaluated by the above organoleptic evaluation criteria 1. The results are given in Table 3.

**Table 3**

| Ingredients | Example5 | Example6 | Example7 | Comp. Example2 |
|---|---|---|---|---|
| Polyethylene glycol (number average molecular weight: 200) | 20.0 | 20.0 | 20.0 | 20.0 |
| Polyethylene glycol (number average molecular weight: 400) | 20.0 | 20.0 | 20.0 | 20.0 |
| Polyoxyethylene glycerol ether *7 | 15.0 | 15.0 | 10.0 | 15.0 |
| Glycerol | 15.0 | 15.0 | 15.0 | 15.0 |
| Synthetic calcined hydrotalcite *8 | 15.0 | 5.0 | 5.0 | - |
| Silicic acid anhydride *9 | - | 10.0 | 5.0 | - |
| Hydroxypropyl cellulose | 0.6 | 0.6 | 0.6 | 0.6 |
| Kaolin | reminder | reminder | reminder | reminder |
| Aluminum starch octenylsuccinate | 2.0 | 2.0 | 2.0 | 2.0 |
| synthetic sodium silicate | 1.0 | 1.0 | 1.0 | 1.0 |
| Sodium polyacrylate (blue colored powder mean particle diameter: 10 µm) | - | 1.0 | 1.0 | - |
| Silicone oil *10 | 0.2 | 0.2 | 5.0 | 0.2 |
| Witch hazel extract (1,3-BG extract) | 1.0 | 1.0 | 1.0 | - |
| Sage leaf extract (1,3-BG extract) | 1.0 | 1.0 | 1.0 | - |
| Asebiol (ASEBIOL BT; trade name) *11 | 1.0 | 1.0 | 1.0 | - |
| Perfume | 0.1 | 0.1 | 0.1 | 0.1 |
| Viscosity(cps, 25°C) | 15,000 | 19,000 | 82,000 | 3,100 |
| Clammy feeling at the time of application | ○ | ⓞ | ⓞ | × |
| Dripping after application | ○ | ⓞ | ⓞ | Δ |
| Warm feeling and durability | ○ | ○ | ⓞ | Δ |

| | | | | |
|---|---|---|---|---|
| *7 UNIOX G-1200 (total E.O.=26, NOF CORPORATION) | | | | |
| *8 KYOWAAD 2000 (powder in the shape of a spherical, Kyowa Chemical Industry Co.,Ltd.) | | | | |
| *9 SYLOPURE 35 (calcined) (Fuji Silysia Chemical Ltd.) | | | | |
| *10 polyoxyethylene-modified organopolysiloxane (Shin-Etsu Chemical Co.,Ltd., silicone KF-351A, number of additional ethylene oxide units=11) | | | | |
| *11 LABORATOIRES SEROBIOLOGIQUES (hydrolyzed yeast/pyridoxine/ niacinamide/glycerol/panthenol/propyleneglycol/allantoin/biotin included) | | | | |

As can be seen from the results given in Table 3, lower column, Examples 5 to 7 according to the present invention indicated durable heat-generating action, no dripping from the skin, and no clammy feeling. Especially Examples 6 and 7 proved excellent. Also, each example showed excellent pack effects giving moisture etc. to the skin compared with the comparative example. Furthermore the heat-generating cosmetic in each example proved safe with no stimulation or trouble observed on any of the panelists' skins after application.

### Example 8

Washable heat-generating packs were prepared according to the formulation shown in Table 4, and the obtained packs were oraganoleptically evaluated by the above organoleptic evaluation criteria 1. The results are given in Table 4.

**Table 4**

| Ingredients | Example8 |
|---|---|
| 1,3-butylene glycol | 40.0 |
| Polyoxyethylene glycerol ether *7 | 15.0 |
| Glycerol | 15.0 |
| Synthetic calcined hydrotalcite *8 | 10.0 |
| Silicic acid anhydride *9 | 5.0 |
| Hydroxypropyl cellulose | 0.6 |
| Kaolin | reminder |
| Aluminum starch octenylsuccinate | 2.0 |
| Synthetic sodium silicate | 1.0 |
| Sodium polyacrylate (mean particle diameter: 10 µm) | 1.0 |
| Silicone oil *10 | 0.2 |
| Witch hazel extract (1,3-BG extract) | 1.0 |
| Sage leaf extract (1,3-BG extract) | 1.0 |
| Asebiol (ASEBIOL BT; trade name) *11 | 1.0 |
| Perfume | 0.1 |
| Clammy feeling at the time of application | ⓞ |
| Dripping after application | ⓞ |
| Warm feeling and durability | ⓞ |

| | |
|---|---|
| (*7 to 11); same as what was given in Examples 5 to 7 | |

### Examples 9 to 10, Comparative Examples 3 to 4

Washable heat-generating packs were prepared according to the formulation shown in Table 5, and the obtained packs were examined oraganoleptically regarding each evaluation point and were evaluated by the following organoleptic evaluation criteria 2. The results are given in Table 5. The durability of heat generated was evaluated by whether the effects sustained for 5 minutes or not.

### [organoleptic evaluation criteria 2]

○ : 7 to 10 persons answered "good"
Δ : 3 to 6 persons answered "good"
× : 0 to 2 persons answered "good"

**Table 5**

| Ingredients | Example9 | Example10 | Comp. Example3 | Comp. Example4 |
|---|---|---|---|---|
| Polyoxyethylene glycerol ether*12 | 50.0 | 50.0 | 50.0 | - |
| Polyoxyethylene methyl glucoside *13 | 1.0 | 1.0 | 1.0 | - |
| Glycerol | - | - | - | 51.0 |
| Zeolite *14 | 38.0 | 38.0 | - | 38.0 |
| Hydroxypropyl cellulose | 0.7 | 0.7 | 0.7 | 0.7 |
| Kaolin | reminder | reminder | reminder | reminder |
| Synthetic magnesium sodium silicate | 1.0 | 1.0 | 1.0 | 1.0 |
| Titanium oxide | 0.4 | 0.4 | 0.4 | 0.4 |
| Talc | 1.0 | 1.0 | 1.0 | 1.0 |
| Sodium polyacrylate (blue colored powder, mean particle diameter: 10 µm) | - | 1.0 | - | - |
| Silicone oil *15 | 0.2 | 0.2 | 0.2 | 0.2 |
| Witch hazel extract (1,3-BG extract) | 1.0 | 1.0 | 1.0 | 1.0 |
| Salvia officinalis extract (1,3-BG extract) | 1.0 | 1.0 | 1.0 | 1.0 |
| Perfume | 0.1 | 0.1 | 0.1 | 0.1 |
| Clammy feeling at the time of application | ○ | ○ | Δ | Δ |
| Warm feeling and durability | ○ | ○ | × | Δ |
| Wash-away ability | ○ | ○ | ○ | × |

| | | | | |
|---|---|---|---|---|
| *12 UNIOX G-1200 (total E.O.=26, NOF CORPORATION) | | | | |
| *13 Glucam E-20 (total E.O.=26, Amerchol Co.) | | | | |
| *14 Zeolum A-4 (TOSOH Corporation) , Na₂O·Al₂O₃·2SiO₂ | | | | |
| *15 polyoxyethylene-modified organopolysiloxane (Shin-Etsu Chemical Co., Ltd., silicone KF-351A, number of additional ethylene oxide units=11) | | | | |

As can be seen from the results given in Table 5 lower column, Examples 9 and 10 according to the present invention indicated durable heat-generating action and little clammy feeling; in particular, Example 2, gave no clammy feeling. Also, Examples 9 and 10 indicated excellent wash-away performance, with no trouble on the skin observed after application, and pack effects on the skin obtained.

### Example 11

Washable heat-generating packs were prepared according to the formulation shown in Table 6, and obtained packs were evaluated by the above organoleptic evaluation method. The results are given in Table 6.

**Table 6**

| Ingredients | Example11 |
|---|---|
| Polyoxyethylene glycerol ether *16 | 50.0 |
| Zeolite *17 | 20.0 |
| Silicic acid anhydride *18 | 5.0 |
| Hydroxypropyl cellulose | 0.6 |
| Kaolin | reminder |
| Aluminum starch octenylsuccinate | 2.0 |
| Synthetic sodium silicate | 1.0 |
| Sodium polyacrylate (mean particle diameter: 10 µm) | 1.0 |
| Silicone oil *19 | 0.2 |
| Witch hazel extract (1,3-BG extract) | 1.0 |
| Sage leaf extract (1,3-BG extract) | 1.0 |
| Vanillyl-n-butylether | 0.05 |
| Perfume | 0.1 |
| Clammy feeling at the time of application | ○ |
| Warm feeling and durability | ○ |
| Wash-away ability | ○ |

| | |
|---|---|
| *16 UNIOX G-1200 (total E.O.=26, NOF CORPORATION) | |
| *17 Zeolum A-3 (Tosoh Corporation) (1-x)Na₂O·xK₂O·Al₂O₃·2SiO₂(x≧ 0.3) | |
| *18 SYLOPURE 35 (calcined)(Fuji Silysia Chemical Ltd. | |
| *19 polyoxyethylene-modified organopolysiloxane (Shin-Etsu Chemical Co.,Ltd., silicone KF-351A, number of additional ethylene oxide units=11) | |

### INDUSTRIAL APPLICABILITY

As described above, the present invention is able to provide a heat-generating cosmetic, whose heat generating action lasts long, which does not drip off from the skin and is excellent in feeling (e.g. giving no clammy feeling), etc.

## Claims

1. A heat-generating cosmetic comprising
(a) a polyhydric alcohol and/or a polyoxyalkylene (having 2 or 3 carbon atoms)-glycol adduct, which generates heat in contact with water,
(b) one or more compounds selected from the group consisting of silicic acid anhydride, silicic acid hydrate, synthetic hydrotalcite and synthetic calcined hydrotalcite, and
(c) a thickening agent,
and which is substantially non-aqueous.

2. A heat-generating cosmetic comprising
(a) a polyoxyalkylene (having 2 or 3 carbon atoms)glycol adduct, which generates heat in contact with water,
(b) zeolite, and
(c) a thickening agent,
and which is substantially non-aqueous.

3. The heat-generating cosmetic according to either Claim 1 or 2, wherein said polyhydric alcohol or polyoxyalkylene (having 2 or 3 carbon atoms)-glycol adduct is at least one compound selected from the group consisting of polyethylene glycol, 1,3-butylene glycol, glycerol, polyoxyethylene glyceryl ether and polyoxyethylene-modified organosiloxane.

4. The heat-generating cosmetic according to either Claim 1 or 2, wherein said thickening agent is at least one compound selected from the group consisting of hydroxypropyl cellulose, aluminum starch octenylsuccinate, synthetic aluminum silicate and kaolin.

5. The heat-generating cosmetic according to any one of Claims 1 to 4, which further comprises (d) sodium polyacrylate powder.
